# EUROPÄISCHE PATENTSCHRIFT

(11) **EP 2 057 103 B1**
(45) Veröffentlichungstag und Bekanntmachung des Hinweises auf die Patenterteilung: **02.07.2014**
(21) Anmeldenummer: 07801216.8
(22) Anmeldetag: 30.07.2007
(51) Int. Cl.: B01J 23/04, B01J 23/58, C04B 33/24, G01N 27/62, G01N 33/00, G01N 30/02, G01N 30/64

(54) **KATALYTISCH AKTIVES BAUELEMENT FÜR THERMOIONISATIONSDETEKTOREN ZUM NACHWEIS VON HALOGENHALTIGEN VERBINDUNGEN UND VERFAHREN ZUR HERSTELLUNG EINES OXIDKERAMISCHEN WERKSTOFFS FÜR DAS BAUELEMENT**
CATALYTICALLY ACTIVE COMPONENT FOR THERMAL IONIZATION DETECTORS FOR THE DETECTION OF HALOGEN-CONTAINING COMPOUNDS AND PROCESS FOR PRODUCING AN OXIDE-CERAMIC MATERIAL FOR THE COMPONENT
COMPOSANT CATALYTIQUEMENT ACTIF POUR DÉTECTEURS DE THERMO-IONISATION EN VUE DE LA DÉTECTION DE COMPOSÉS HALOGÉNÉS ET PROCÉDÉ DE FABRICATION D'UN MATÉRIAU DE CÉRAMIQUE D'OXYDE POUR LE COMPOSANT

(30) Priorität: 29.08.2006 DE 102006041510
(43) Veröffentlichungstag der Anmeldung: 13.05.2009
(73) Patentinhaber: Fraunhofer-Gesellschaft zur Förderung der angewandten Forschung e.V., 80686 München (DE)
(72) Erfinder: SAUCHUK, Viktar, 01069 Dresden (DE); OTSCHIK, Peter, 01728 Possendorf (DE); EICHLER, Klaus, 01187 Dresden (DE); KUSNEZOFF, Mihails, 01159 Dresden (DE)
(74) Vertreter: Pfenning, Meinig & Partner GbR
(86) Internationale Anmeldenummer: PCT/DE2007/001405
(87) Internationale Veröffentlichungsnummer: WO 2008/025320

(56) Entgegenhaltungen:
- GB-A- 2 261 534
- US-A- 2 795 716
- US-A- 4 839 143
- US-A- 4 928 033
- US-A- 5 521 098
- KIM S I ET AL: "CESIUM ION TRANSPORT ACROSS A SOLID ELECTROLYTE-POROUS TUNGSTEN INTERFACE" JOURNAL OF VACUUM SCIENCE AND TECHNOLOGY: PART A, AVS /AIP, MELVILLE, NY, US, Bd. 7, Nr. 311, 1. Mai 1989 (1989-05-01), Seiten 1806-1809, XP000045608 ISSN: 0734-2101

## Beschreibung

Die Erfindung betrifft katalytisch aktive Bauelemente für Thermoionisationsdetektoren zum Nachweis von halogenhaltigen Verbindungen und ein Verfahren zur Herstellung eines oxidkeramischen Werkstoffs für diese Bauelemente. Dabei ist ein Bauelement aus einem oxidkeramischen Sinterwerkstoff gebildet und für die Anwendung in hochempfindlichen Ionisationsdetektoren geeignet. Der oxidkeramische Sinterwerkstoff weist ein verbessertes Gefüge gegenüber herkömmlichen technischen Lösungen auf.

Das erfindungsgemäße Bauelement ist insbesondere vorteilhaft für Detektoren in der Gaschromatographie, wobei der Werkstoff als katalytisch aktives Bauelement eines selektiven Ionisationsdetektors zum Nachweis von halogenhaltigen Verbindungen besonders geeignet ist. Der Werkstoff wird bei hohen Temperaturen im Bereich zwischen 700 und 1000 °C eingesetzt. Er ist dabei chemisch und mechanisch stabil und auch für den erforderlichen Langzeitbetrieb geeignet.

Ein Ionisationsdetektor ist ein analytisches Nachweisgerät, in dem gasförmige Komponenten bei erhöhter Temperatur ionisiert und durch den entstehenden Ionenstrom detektiert werden.

Die Natur des Ionisierungsmechanismus, der in diesen Vorrichtungen abläuft, ist bisher nicht eindeutig geklärt. Eine Theorie geht dahin, dass die Ionisation des nachzuweisenden Stoffes an der Oberfläche des katalytisch aktiven Bauelements bei erhöhter Temperatur stattfindet. Das katalytisch aktive Bauelement des Detektors ist ein spezieller Werkstoff, der Alkaliatome enthält. Es wird dabei angenommen, dass die Alkaliatome als katalytisch aktive Zentren wirken, die die Ionisation des nachweisenden Stoffes verursachen.

Aus dem Stand der Technik sind unterschiedliche Werkstoffe und Herstellungstechnologien für aktive Elemente in selektiven Ionisationsdetektoren bekannt.

So ist es bekannt (P.L. Patterson, US-Patent 4203726, 05/1980), Tonerdekeramik mit den darin enthaltenen natürlichen Alkaliverunreinigungen zu verwenden. Mit diesem Werkstoff ergibt sich aber oft das Problem, dass die erforderliche Sensoreigenschaft nur für eine relativ kurze Betriebsdauer möglich ist. Es wird versucht, diesem Nachteil dadurch entgegen zu wirken, dass die Tonerde mit einer wässrigen Lösung, die ein Alkalimetallsalz enthielt, getränkt wird.

Diese synthetischen alkalihaltigen Verbindungen können zu einer verlängerten Betriebsdauer des Elements führen. So werden synthetische Alkalialuminiumsilikatgläser wie beispielsweise Li₂O:Al₂O₃:SiO₂= 1:1:2 verwendet, um positive Ionen in Ionisationsdetektoren zu erzeugen. Das gelingt insbesondere, wenn der Werkstoff auf Temperaturen in der Nähe des Schmelzpunktes des Alkaliglases erhitzt wird (J.P. Blewett, E.J. Jones "Filament Sources of Positive Iones"; Pysical Review, 50 (1936) 484).

Im Jahre 1951 hat C.W. Rice (US-Patent 2550498) eine Vorrichtung zum elektrischen Nachweis von Dämpfen gewisser Substanzen beschrieben, bei der als Werkstoff Natrium- und Kaliumsilikate Na₂SiO₃ und K₂SiO₃ sowie synthetische Leucite Li₂O:Al₂O₃:SiO₂=1:1:2 vorgeschlagen werden.

Alkalisulfate wurden zur Herstellung der alkalihaltigen aktiven Elemente als besonders geeignet gefunden. Andere Arten von Alkaliverbindungen, die verwendet wurden, sind Alkalikarbonate, AlkaliHalogenide und Alkaliiodide.

Zum spezifischen Detektieren von Stickstoff- und Phosphor-Verbindungen wurden Alkali-Keramik-Mischungen eingesetzt, die aus 6 Gew.-% Rb₂SO₄ und 94 Gew.-% Keramikzement (1st Super Refractory Cement C-10 der Firma Dylon Industries, Inc., Cleveland, Ohio, USA) bestehen (P.L. Patterson, US-Patent 4203726, 05/1980).

Ein wesentlicher Nachteil von Detektoren mit derartigen katalytisch aktiven Werkstoffen besteht in einer ungenügenden Empfindlichkeit, was ihren Betrieb und ihre Einsatzmöglichkeiten begrenzt.

Um diesen Nachteil dieser aktiven Elemente entgegenzuwirken, wurden Werkstoffe mit mehreren unterschiedlichen Alkalimetallen eingesetzt.

Es wird ferner angenommen, dass die Menge der Alkalimetalle in der Keramik von der vorgesehenen Verwendung des aktiven Keramikelements abhängt und demzufolge von Spurkonzentrationen bis zu 40 Gew.-% variiert werden kann (R.P. White, B.W. Hermann, R.D. Worden, US-Patent 5498548, 03/1996).

P.L. Patterson hat in seinem Thermoionisationsdetektor eine Mehrschichtionisationsquelle verwendet, um sowohl eine lange Betriebsdauer als auch eine hohe Empfindlichkeit des Sensors zu gewährleisten (P.L. Patterson, US-Patent 4524047, 06/1985). Dabei werden mehrere Schichten auf einem Heizelement ausgebildet, die jeweils eine unterschiedliche Zusammensetzung aufweisen, um unterschiedliche Funktionen zu erfüllen. Ein solcher Mehrschichtaufbau erhöht den Herstellungsaufwand und bereitet bei wechselnden Temperaturen Probleme bzgl. der Haftung und Eigenspannungen.

Die erhöhte Konzentration von Alkalimetallen im Werkstoff hat oft den Nachteil, dass diese Metalle in der Matrix unkontrolliert und nicht homogen verteilt sind.

Außerdem resultiert eine Erhöhung des Alkalimetallgehalts gewöhnlich in einer Herabsetzung des Schmelzpunktes und infolgedessen auch verstärkter Alkali-Abdampfung aus der Oberfläche des Werkstoffes. Dementsprechend sind aktive Elemente mit höheren Alkaligehalten bei ihrer Verwendung bei hohen Temperaturen durch den Beginn des Schmelzens begrenzt.

Die Erhöhung der Alkalimetallkonzentration im Werkstoff ruft auch eine Herabsetzung der mechanischen Festigkeit und damit der Lebensdauer des Elements hervor.

Außerdem ist es oft schwierig die synthetischen alkalihaltigen Werkstoffe in gleicher Qualität herzustellen, so dass eine Reproduzierbarkeit der Ergebnisse beim Einsatz mehrerer Detektoren nicht zuverlässig gewährleistet werden kann.

Aus US 2, 795, 716 A ist ein elektrischer Dampfdetektor bekannt, bei dem ein alkalisches Metallglas eingesetzt wird.

Es ist daher Aufgabe der Erfindung, ein katalytisch aktives Bauelement für Thermoionisationsdetektoren zum Nachweis von halogenhaltigen Verbindungen zur Verfügung zustellen, das thermisch, mechanisch und chemisch dauerhaft stabil ist und eine erhöhte Empfindlichkeit zu den nachweisenden Stoffen aufweist.

Der für die Bauelemente eingesetzte Werkstoff soll dabei in dem die für den Detektor notwendigen optimalen Parameter kontrollierbar einstellbar sein. Eine weitere Aufgabe besteht darin, ein entsprechendes Herstellungsverfahren anzugeben.

In Bezug auf das katalytisch aktive Bauelement wird die Aufgabe durch die Merkmale des Patentanspruchs 1 und in Bezug auf das Verfahren zur Herstellung des Werkstoffs durch die Merkmale des Patentanspruchs 8 gelöst. Die Unteransprüche zeigen vorteilhafte Weiterbildungen auf.

Erfindungsgemäß wird somit vorgeschlagen, ein mit oxidkeramischem Sinterwerkstoff gebildetes katalytisch aktives Bauelement für Thermoionisationsdetektoren zur Verfügung zu stellen, das aus einer kristallinen Phase und einer amorphen Glasphase besteht, wobei es erfindungswesentlich ist, dass die amorphe Glasphase mit 0,1 bis 20 Gew.-% einer Cäsiumverbindung gebildet ist.

Es hat sich überraschenderweise herausgestellt, dass durch das Zumischen der Cäsiumverbindung eine deutliche Erhöhung der Empfindlichkeit eines entsprechenden Detektors, der aus dem oxidkeramischen Sinterwerkstoff nach der Erfindung hergestellt wird, erreicht wird.

Es hat sich weiterhin gezeigt, dass dann, wenn der Gehalt an der Cäsiumverbindung, die der oxidkeramische Sinterwerkstoff enthält, über 20 Gew.-% steigt, eine Verschlechterung der mechanischen Festigkeit des Werkstoffs bei den erforderlichen Betriebstemperaturen eintritt, so dass ein höherer Cäsiumgehalt nachteilig ist. Bei Gehalten der Cäsiumverbindung unter 0,1 Gew.-% werden keine signifikanten Steigerungen in der Empfindlichkeit erreicht, so dass eine Untergrenze von 0,1 wesentlich ist. Es hat sich weiterhin gezeigt, dass auch dann, wenn der oxidkeramische Werkstoff nur bis zu 10 Gew.-% einer Cäsiumverbindung enthält, noch brauchbare Eigenschaften in Bezug auf die mechanische Festigkeit des Werkstoffs bei den Betriebstemperaturen erzielt werden, so dass diese Obergrenze für den erfindungsgemäß eingesetzten Werkstoff bevorzugt ist.

Zur weiteren Verbesserung der mechanischen Festigkeit werden weiter Partikel aus Platin mit einer Primärpartikelgroße im Nanobereich zugemischt. Dadurch kann die Lebensdauer auch bei Einhaltung einer erhöhten Empfindlichkeit verlängert werden. Während des normalen Betriebs eines Detektors verarmt die Oberfläche des Bauelements an aktiven katalytischen Alkalizentren während des Dauerbetriebs bei den erhöhten Einsatztemperaturen in Folge einer Abdampfung. Demzufolge reduziert sich die Empfindlichkeit der Detektoren über die Betriebszeit. Dies kann durch die aktivierende Wirkung der zusätzlichen Platinnanopartikel verhindert zumindest jedoch reduziert werden.

Da Platin chemisch neutral ist werden während des Sinterprozesses keine Verbindungen mit dem Platin gebildet.

Die homogen verteilten Nanopartikel aus Platin bilden Störstellen in der gesinterten Keramikstruktur. Diese Störstellen begünstigen die Wanderung der leicht beweglichen Alkali-Ionen durch den Werkstoff des Bauelements zu dessen Oberfläche. So können beim Betrieb an der Oberfläche des Bauelements Alkali-Ionen, die vorab entfernt worden sind, ersetzt werden. So kann die Lebensdauer bei Einhaltung einer ausreichenden Detektionsempfindlichkeit von Thermoionisationsdetektoren, durch die mögliche Zufuhr von Cäsium-Ionen an die Oberfläche des Bauelements, verlängert werden.

Beim erfindungsgemäß für das katalytische aktive Bauelement eingesetzten Werkstoff ist die kristalline Phase bevorzugt aus Quarz (SiO₂), Aluminiumoxid (Al₂O₃) und Mullit (2SiO₂3Al₂O₃) gebildet. Eine Bildung von CsAlSiO₄ oder CsAlSi₂O₆ als eine Nebenphase ist auch möglich.

Es kann ein Anteil an kristalliner Phase bis zu 60 Vol.-% vorhanden sein. Ein höherer Anteil würde zu einer Reduzierung der Empfindlichkeit/Sensitivität bei der Detektion führen.

Bei den Cäsiumverbindungen sind insbesondere die oxidischen Verbindungen des Cäsiums bevorzugt und hier Cs₂O. Die Erfindung umfasst aber auch bei den oxidischen Verbindungen des Cäsiums solche mit einer abweichenden Oxidationsstufe von Cs₂O.

Die Erfindung bietet weiterhin ein Verfahren zur Herstellung des oxidkeramischen Sinterwerkstoffs für das katalytisch aktive Bauelement.

Erfindungsgemäß wird dabei so vorgegangen, dass in einer ersten Stufe eine getrocknete Tonerdeporzellankeramikmasse in Pulverform mit einer wässrigen Lösung der Cäsiumverbindung hergestellt wird. Nach Trocknung dieser Masse wird diese dann einer Formgebung unterzogen und bei einer Temperatur von 1100 bis 1400 °C gesintert.

Beim Herstellungsverfahren ist es dabei besonders wichtig, dass die definierten Sintertemperaturen eingehalten werden, da bei tiefen Temperaturen, d.h. unter 1100 °C, die zu sinternde Masse spröde wird und der gesinterte Formkörper eine geringe Empfindlichkeit und eine schlechte Reproduzierbarkeit der Messergebnisse zeigt. Mit steigender Temperatur der Sinterbehandlung der Rohmasse verbessern sich deren mechanische Stabilität und die Ionisationseigenschaften des Detektors. Die Empfindlichkeit des Detektors steigt mit der Temperatur der Thermobehandlung allerdings nur bis zu einer Sintertemperatur von 1400 °C an. Höhere Temperaturen haben sich als ungünstig erwiesen. Es hat sich gezeigt, dass es besonders günstig ist, wenn die Thermobehandlung bei 1210 °C ± 10 °C durchgeführt wird.

Bei Temperaturen oberhalb 1210 °C sind die Stoffumwandlungsprozesse im Werkstoff abgeschlossen und es steigt dann der Anteil an Kristallphase im Werkstoff weiter an. Dadurch werden die Alkali-Ionen im Werkstoff fest eingebunden, wie z.B. Cäsiumionen in hochschmelzendem Polluzit CsAlSi₂O₆.

Dadurch reduziert sich die katalytische Aktivität. Bei steigender Sintertemperatur steigt aber auch der Dampfdruck der leichtflüchtigen Alkalikomponenten, wodurch die Konzentration der Alkaliatome und demzufolge auch die katalytische Aktivität zusätzlich reduziert werden.

Aus stofflicher Sicht umfasst das erfindungsgemäße Verfahren in Bezug auf die Tonerdeporzellankeramikmassen, die in Pulverform eingesetzt werden, alle an und für sich im Stand der Technik bekannten Keramikmassen, so insbesondere Ausgangsmaterialien, wie Feldspat, Kaolin und Tonerde. Bei den Cäsiumverbindungen sind insbesondere Cäsiumkarbonat, d.h. eine wässrige Lösung von Cäsiumkarbonat bevorzugt.

Die Gewichtsverhältnisse, in denen die Tonerdeporzellankeramikmassen und die Cäsiumverbindung eingesetzt werden, sind bevorzugt für die Tonerdeporzellankeramikmassen 80 Gew.-% bis 99,9 Gew.-% und für die Cäsiumverbindung 0,1 bis 20 Gew.-% bezogen auf die Gesamtmasse der Mischung. Platinnanopartikel sollen mit einem Anteil von 5 bis 10 Gew.-% bezogen auf die trockene Masse (gesamte Ausgangsmasse) der eingesetzten Tonerdeporzellankeramik und der Cäsiumverbindung eingesetzt werden. Die eingesetzten Pulver sollten eine mittlere Primärpartikelgröße von 50 nm bis 100 nm aufweisen.

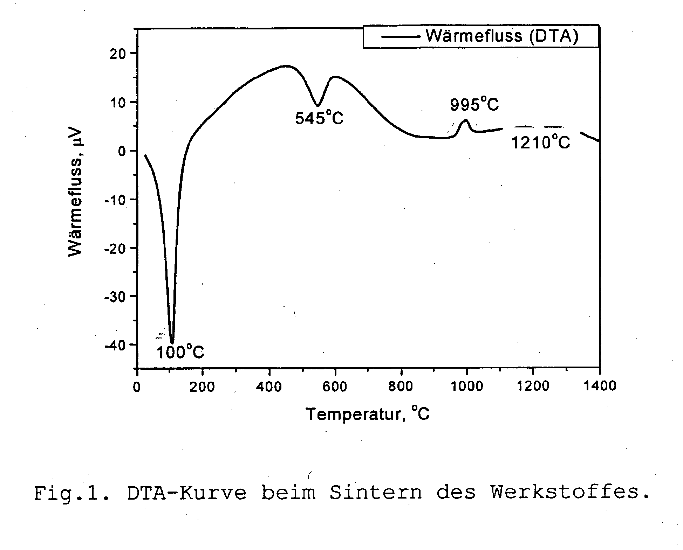

Die Erfindung wird nachfolgend anhand eines Ausführungsbeispiels und den Figuren 1 bis 6 näher beschrieben.

Ausführungsbeispiel 1: Herstellung eines oxidkeramischen Sinterwerkstoffs für katalytisch aktive Bauelemente:
Es werden 5,78 g Cs₂CO₃, die der Menge von 5 g Cs₂O entsprechen eingewogen und in 50 ml deionisiertem Wasser durch Rühren gelöst. Diese Lösung wird mit 95 g eines getrockneten Keramikrohstoffpulvers C130 (Hersteller DWS-Keramik Neuhaus GmbH, Neuhaus Schiernitz, DE) gemischt und durch sorgfältiges Rühren homogenisiert. Das homogene Material wird bei Raumtemperatur getrocknet.

Ausführungsbeispiel 2: Herstellung eines mit Platin zusätzlich aktivierten erfindungsgemäßen oxidkeramischen Sinterwerkstoffs für katalytisch aktive Bauelemente:
95 g getrocknetes Keramikrohstoffpulver C130 werden mit 5,263 g pulverförmigen Platin in einem Mischer gemischt. Das Gemisch wird in 50 ml deiniosiertes Wasser und 5,78 g darin gelöstes Cs₂CO₃ gegeben, gerührt und das Gemisch homogenisiert. Das Gemisch wurde bei Raumtemperatur getrocknet. Die Sinterung erfolgte dann bis eine maximale Sintertemperatur von 1210 °C erreicht wurde.

- Figur 1: zeigt einen DTA-Verlauf beim Sinterprozess;
- Figur 2: zeigt ein Röntgendiffraktogramm eines erfindungsgemäß gesinterten Werkstoffs;
- Figur 3: zeigt Messergebnisse bei verschiedenen Sintertemperaturen des Werkstoffs;
- Figur 4: zeigt eine EDX-Analyse;
- Figur 5: zeigt ein Diagramm der Empfindlichkeit des erfindungsgemäßen Werkstoffs als Ionisationsdetektor im Vergleich zum Stand der Technik und
- Figur 6: zeigt die Empfindlichkeit eines erfindungsgemäßen Detektors für verschiedene Zusammensetzungen des Werkstoffs.

Mit dem in Figur 1 gezeigten DTA-Verlauf kann verdeutlicht werden, dass die Umwandlunsgprozesse im Werkstoff für das Bauelement ausgehend vom Rohwerkstoff bis zu einer Temperatur von 1210 °C ablaufen, so dass die Wärmebehandlung beim Sintern bei einer Temperatur im engen Temperaturbereich 1210 °C ± 10 °C besonders günstig ist.

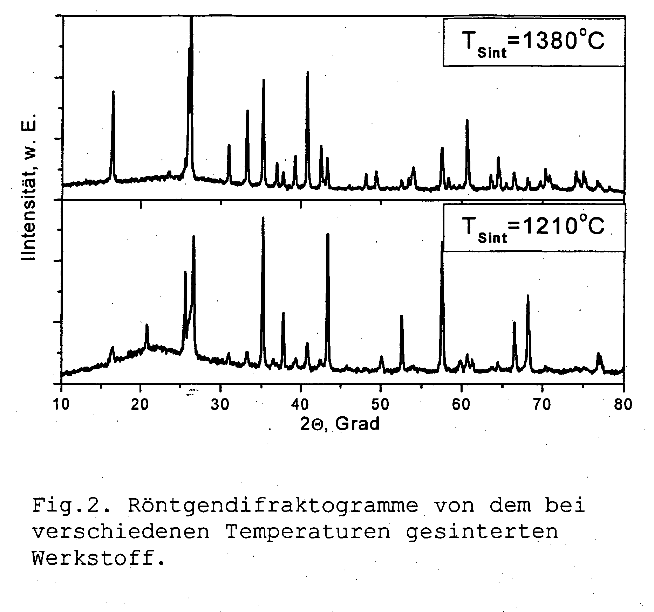

Das Röntgendiffraktogramm des gesinterten Werkstoffes in Figur 2 zeigt die kristallinen Phasen Quarz (SiO₂), Aluminiumoxid (Al₂O₃) und Mullit (2SiO₂3Al₂O₃) sowie einen erheblichen Anteil von Glasphase, die für die Funktion des Bauelements wichtig ist.

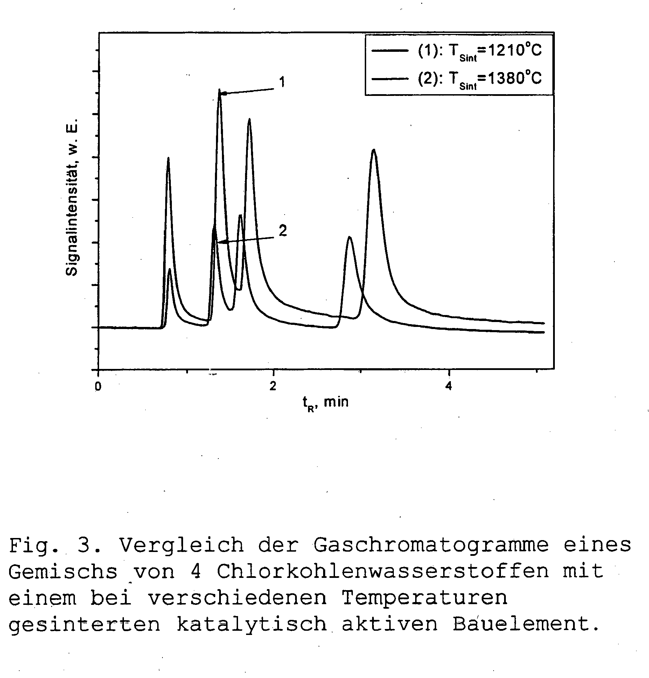

Die Unterschiede in der Empfindlichkeit des Detektors mit den bei verschiedenen Temperaturen gesinterten aktiven Bauelementen sind in Figur 3 deutlich erkennbar. Es folgt auch aus Figur 3, dass die optimale Sintertemperatur des katalytisch aktiven Bauelements des Detektors bei 1210°C liegt.

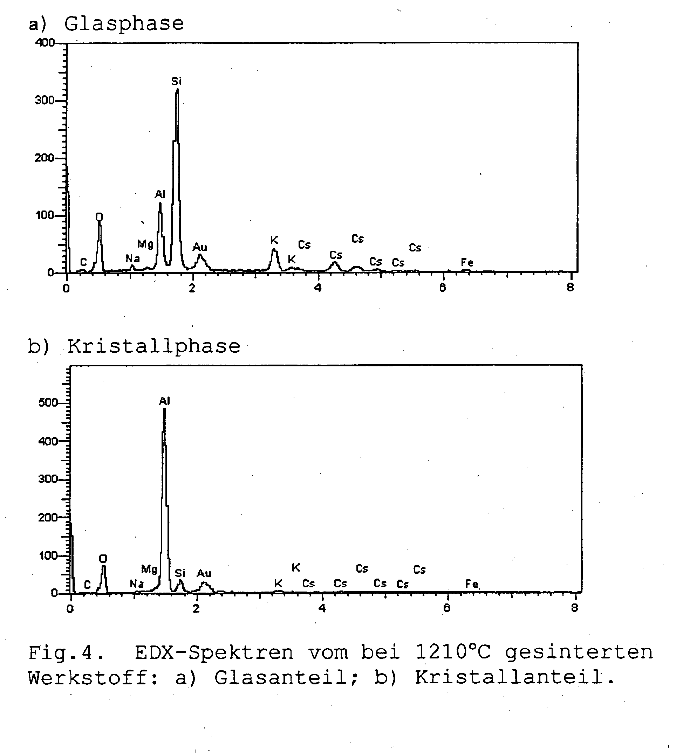

Aus der in Figur 4 gezeigten EDX-Analyse wird deutlicht, dass die Alkalimetalle vorwiegend in der Glasphase des gesinterten Werkstoffes verteilt sind, so dass die katalytische Aktivität des Werkstoffes hauptsächlich durch den Glasanteil gewährleistet ist.

Eine Zumischung von Cäsium zur Tonerdeporzellanmasse verursacht eine deutliche Erhöhung von Empfindlichkeit eines Detektors mit katalytisch aktivem Bauelement.

Der erfindungsgemäß eingesetzte Werkstoff wird aus einer getrockneten Tonerdeporzellankeramikmasse in Pulverform und einer wässrigen Lösung von Cäsiumcarbonat hergestellt. Dazu wird die wässrigen Lösung von Cäsiumcarbonat mit der Tonerdeporzellankeramikmasse zu einer breiförmigen Masse verarbeitet, sorgfältig homogenisiert, bei der Raumtemperatur getrocknet und geformt und schließlich bei einer Temperatur von 1210±10°C gesintert.

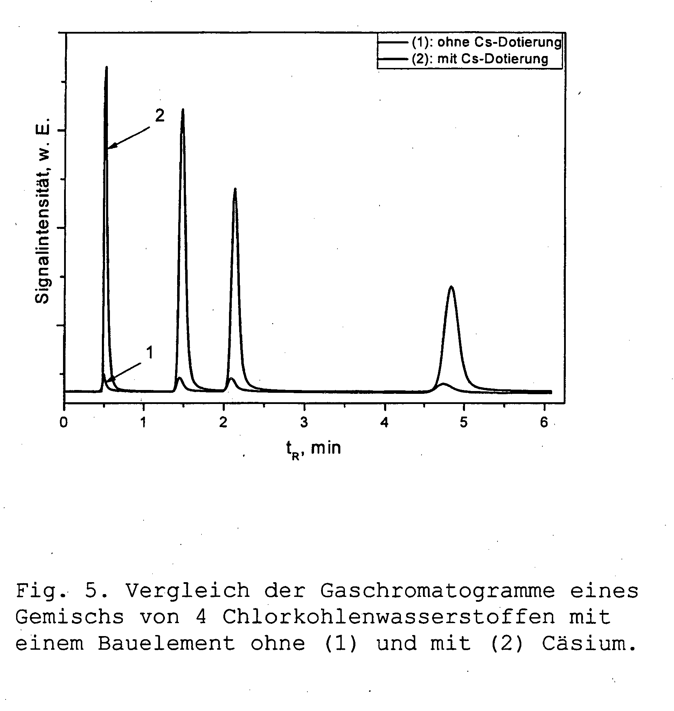

Das Diagramm in Figur 5 zeigt, dass der erfindungsgemäß hergestellte Werkstoff in einem Ionisationsdetektor eine 100-fach größere Empfindlichkeit zu Halogenierten Kohlenwasserstoffen aufweist als Vergleichsdetektoren auf dem gleichen Wirkprinzip. Dabei weist er die gleiche Empfindlichkeit für Halogenierte Kohlenwasserstoffe mit unterschiedlicher Anzahl der Halogenatome auf. Dies deutet darauf hin, dass bei der Ionisation nur einfach geladene Ionen in der Gasphase entstehen.

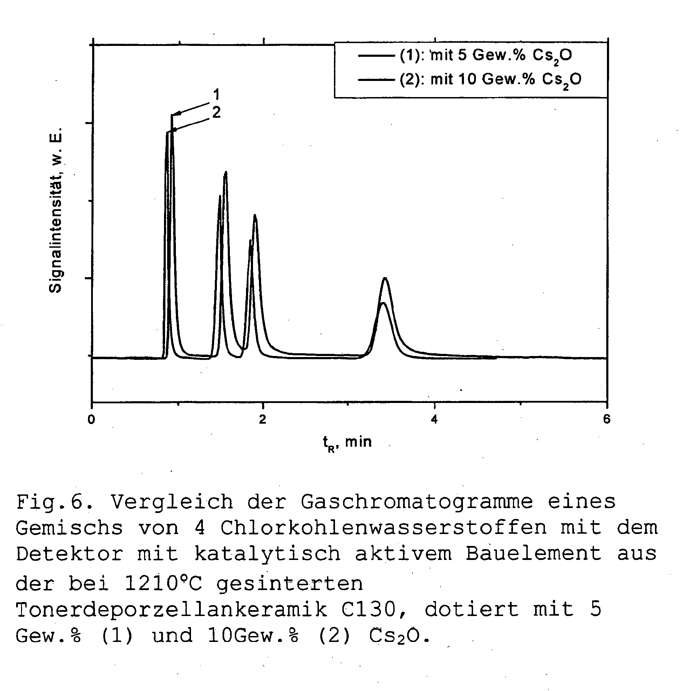

Figur 6 zeigt, dass die Empfindlichkeit eines Detektors mit dem katalytisch aktiven Bauelement für die Zusammensetzungen (C130)_{0.95}(Cs₂O)_{0.05} und (C130)_{0.9}(Cs₂O)_{0.1} vergleichbar ist.

Eine Erhöhung des Cs₂O-Gehaltes über 10 Gew.-% resultiert in einer Verschlechterung der mechanischen Festigkeit des Werkstoffes bei Betriebstemperaturen und ist deshalb nachteilig.

## Patentansprüche

1. Katalytisch aktives Bauelement für Thermoionisationsdetektoren zum Nachweis von halogenhaltigen Verbindungen,
**dadurch gekennzeichnet, dass** das Bauelement aus einem oxidkeramischen Sinterwerkstoff gebildet ist, der aus einer kristallinen und einer amorphen Glasphase besteht, wobei die amorphe Glasphase mit 0,1 bis 20 Gew.-% einer Cäsiumverbindung gebildet ist und Platinpartikel mit einem Anteil von 5 bis 10 Gew.-% enthalten sind.

2. Bauelement nach Anspruch 1, **dadurch gekennzeichnet, dass** die amorphe Glasphase mit 0,1 bis 10 Gew.-% der Cäsiumverbindung gebildet ist.

3. Bauelement nach Anspruch 1 oder 2, **dadurch gekennzeichnet, dass** die Cäsiumverbindung eine oxidische Verbindung des Cäsiums ist.

4. Bauelement nach Anspruch 3, **dadurch gekennzeichnet, dass** die oxidische Verbindung von Cäsium Cs₂O ist.

5. Bauelement nach mindestens einem der Ansprüche 1 bis 4, **dadurch gekennzeichnet, dass** die kristalline Phase durch Quarz (SiO₂), Korund (Al₂O₃), Mullit (2SiO₂*3Al₂O₃), CsAlSiO₄ und/oder CsAlSi₂O₆ gebildet ist.

6. Bauelement nach mindestens einem der Ansprüche 1 bis 5, **dadurch gekennzeichnet, dass** er Zusatzstoffe, wie Flussmittel, Plastifizierer, Verflüssiger und/oder sonstige Hilfsmittel enthält.

7. Bauelement nach einem der vorhergehenden Ansprüche, **dadurch gekennzeichnet, dass** die Platinpartikel eine mittlere Primärpartikelgröße von 50 nm bis 100 nm aufweisen.

8. Verfahren zur Herstellung eines oxidkeramischen Werkstoffs für katalytische aktive Bauelemente von Thermoionisationsdetektoren nach mindestens einem der Ansprüche 1 bis 7 mit folgenden Schritten:
a) Herstellung einer Mischung aus einer pulverförmigen Tonerdeporzellankeramikmasse, einer wässrigen Lösung einer Cäsiumverbindung und 5 bis 10 Gew.-% Platinnanopartikel bezogen auf die Ausgangsmasse von Tonerdeporzellankeramikmasse und Cäsiumverbindung eingesetzt werden,
b) Trocknen der Masse,
c) Formgebung und Sinterung der Masse bei 1100 °C bis 1400 °C.

9. Verfahren nach Anspruch 8, **dadurch gekennzeichnet, dass** die Sinterung bei einer Temperatur von 1210±10 °C durchgeführt wird.

10. Verfahren nach Anspruch 8 oder 9, **dadurch gekennzeichnet, dass** die Trocknung (Verfahrensschritt b) bei Raumtemperatur vorgenommen wird.

11. Verfahren nach mindestens einen der Ansprüche 8 bis 10, **dadurch gekennzeichnet, dass** die Tonerdeporzellankeramikmassen ausgewählt sind aus Feldspat, Kaolin und/oder Tonerde.

12. Verfahren nach mindestens einem der Ansprüche 8 bis 11, **dadurch gekennzeichnet, dass** als Cäsiumverbindung eine wässrige Lösung von Cäsiumkarbonat eingesetzt wird.

13. Verfahren nach mindestens einem der Ansprüche 8 bis 12, **dadurch gekennzeichnet, dass** beim Verfahrensschritt a) 80 bis 99,9 Gew.-% an pulverförmiger Tonerdeporzellankeramikmasse und 0,1 bis 20 Gew.-% der Cäsiumverbindung eingesetzt werden.

## Claims

1. A catalytically active component for thermoionic detectors for detecting halogen-containing compounds,
**characterised in that**
the component is formed from an oxide ceramic sintering material which consists of a crystalline and an amorphous vitreous phase, the amorphous vitreous phase being formed with 0.1 to 20% by weight of a caesium compound and platinum particles in a proportion of 5 to 10% by weight being contained therein.

2. A component according to Claim 1, **characterised in that** the amorphous vitreous phase is formed with 0.1 to 10% by weight of the caesium compound.

3. A component according to Claim 1 or 2, **characterised in that** the caesium compound is an oxidic compound of caesium.

4. A component according to Claim 3, **characterised in that** the oxidic compound of caesium is Cs₂O.

5. A component according to at least one of Claims 1 to 4, **characterised in that** the crystalline phase is formed by quartz (SiO₂), corundum (Al₂O₃), mullite (2SiO₂*3Al₂O₃), CsAlSiO₄ and/or CsAlSi₂O₆.

6. A component according to at least one of Claims 1 to 5, **characterised in that** it contains additives, such as fluxes, plasticisers, liquefiers and/or other auxiliaries.

7. A component according to one of the preceding claims, **characterised in that** the platinum particles have an average primary particle size of 50 nm to 100 nm.

8. A method for the production of an oxide ceramic material for catalytic active components of thermoionic detectors according to at least one of Claims 1 to 7, with the following steps:
a) production of a mixture of a powdered alumina porcelain ceramic mass, an aqueous solution of a caesium compound, and 5 to 10% by weight platinum nanoparticles relative to the starting weight of alumina porcelain ceramic mass and caesium compound is used,
b) drying of the mass,
c) shaping and sintering of the mass at 1100°C to 1400°C.

9. A method according to Claim 8, **characterised in that** the sintering is carried out at a temperature of 1210+10°C.

10. A method according to Claim 8 or 9, **characterised in that** the drying (method step b) is performed at room temperature.

11. A method according to at least one of Claims 8 to 10, **characterised in that** the alumina porcelain ceramic masses are selected from feldspar, kaolin and/or alumina.

12. A method according to at least one of Claims 8 to 11, **characterised in that** an aqueous solution of caesium carbonate is used as caesium compound.

13. A method according to at least one of Claims 8 to 12, **characterised in that** in method step a) 80 to 99.9% by weight of powdered alumina porcelain ceramic mass and 0.1 to 20% by weight of the caesium compound are used.

## Revendications

1. Composant catalytiquement actif pour détecteurs à thermo-ionisation pour la détection de composés halogénés, **caractérisé en ce que** le composant est formé d'un matériau fritté en céramique à base d'oxydes, qui est constitué d'une phase vitreuse cristalline et d'une phase vitreuse amorphe, la phase vitreuse amorphe étant formée dans une proportion de 0,1 à 20 % en poids d'un composé du césium, et **en ce que** le composant contient des particules de platine en une quantité de 5 à 10 % en poids.

2. Composant selon la revendication 1, **caractérisé en ce que** la phase vitreuse amorphe est formée dans un représentation de 0,1 à 10 % en poids du composé de césium.

3. Composant selon la revendication 1 ou 2, **caractérisé en ce que** le composé de césium est un composé de type oxyde de césium.

4. Composant selon la revendication 3, **caractérisé en ce que** le composé de type oxyde de césium est Cs₂O.

5. Composant selon au moins l'une des revendications 1 à 4, **caractérisé en ce que** la phase cristalline est formée de quartz (SiO₂), de corindon (Al₂O₃), de mullite (2SiO₂.3Al₂O₃), de CsAlSiO₄ et/ou de CsAlSi₂O₆.

6. Composant selon au moins l'une des revendications 1 à 5, **caractérisé en ce qu'**il contient des additifs tels que des fondants, des plastifiants, des fluidisants et/ou d'autres adjuvants.

7. Composant selon l'une des revendications précédentes, **caractérisé en ce que** les particules de platine présentent une taille de particule primaire moyenne de 50 nm à 100 nm.

8. Procédé de fabrication d'un matériau céramique à base d'oxydes pour des composants catalytiques actifs de détecteurs à thermo-ionisation selon au moins l'une des revendications 1 à 7, comportant les étapes suivantes :
a) fabrication d'un mélange d'une masse céramique pulvérulente de porcelaine d'alumine, d'une solution aqueuse d'un composé du césium et de 5 à 10 % en poids de nanoparticules de platine, par rapport à la masse de départ de la masse céramique de porcelaine d'alumine et du composé du césium,
b) séchage de la masse,
c) façonnage et frittage de la masse à une température allant de 1100°C à 1400°C.

9. Procédé selon la revendication 8, **caractérisé en ce que** le frittage est mis en oeuvre à une température de 1210 ± 10°C.

10. Procédé selon la revendication 8 ou 9, **caractérisé en ce que** le séchage (étape b) du procédé) est réalisé à la température ambiante.

11. Procédé selon au moins l'une des revendications 8 à 10, **caractérisé en ce que** les masses céramiques à base de porcelaine d'alumine sont choisies parmi le feldspath, le kaolin et/ou l'alumine.

12. Procédé selon au moins l'une des revendications 8 à 11, **caractérisé en ce qu'**on utilise en tant que composé du césium une solution aqueuse de carbonate de césium.

13. Procédé selon au moins l'une des revendications 8 à 12, **caractérisé en ce qu'**on utilise dans l'étape a) du procédé 80 à 99,9 % en poids de la masse céramique pulvérulente à base de porcelaine d'alumine et 0,1 à 20 % en poids du composé du césium.
